(19) Europäisches Patentamt
European Patent Office
Office européen des brevets

(11) **EP 3 017 754 B1**

(12) **FASCICULE DE BREVET EUROPEEN**

(45) Date de publication et mention
de la délivrance du brevet:
**01.12.2021 Bulletin 2021/48**

(51) Int Cl.:
*A61B 5/00* (2006.01)     *A61B 5/024* (2006.01)
*A61B 5/352* (2021.01)

(21) Numéro de dépôt: **15192008.9**

(22) Date de dépôt: **29.10.2015**

(54) **METHODE DE DETERMINATION AUTOMATISEE D'UN INDICE REPRESENTATIF DE L'ACTIVITE SYMPATHO-VAGALE D'UN ETRE HUMAIN OU D'UN ANIMAL DE COMPETITION**

METHODE ZUR AUTOMATISCHEN BESTIMMUNG EINES REPRÄSENTATIVEN INDEXES FÜR DIE SYMPATHO-VAGALE AKTIVITÄT EINES MENSCHEN ODER EINES TIERES IN EINEM WETTKAMPF

METHOD FOR AUTOMATED DETERMINATION OF AN INDEX REPRESENTING THE SYMPATHOVAGAL ACTIVITY OF A HUMAN BEING OR A COMPETITION ANIMAL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.11.2014 FR 1460785**

(43) Date de publication de la demande:
**11.05.2016 Bulletin 2016/19**

(73) Titulaire: **BE INVEST International S.A.**
**3372 Leudelange (LU)**

(72) Inventeur: **SABOUL, Damien**
**01130 Le Poizat (FR)**

(74) Mandataire: **Marks & Clerk France**
**Immeuble "Visium"**
**22, avenue Aristide Briand**
**94117 Arcueil Cedex (FR)**

(56) Documents cités:
- **"Heart rate variability. Standards of measurement, physiological interpretation, and clinical use. Task Force of the European Society of Cardiology and the North American Society of Pacing and Electrophysiology", EUROPEAN HEART JOURNAL, OXFORD UNIVERSITY PRESS, GB, vol. 17, no. 3, 1 mars 1996 (1996-03-01), pages 354-381, XP002342325, ISSN: 0195-668X**
- **PORTA A ET AL: "Non-invasive model-based estimation of the sinus node dynamic properties from spontaneous cardiovascular variability series", MEDICAL & BIOLOGICAL ENGINEERING & COMPUTING, SPRINGER, BERLIN, DE, vol. 41, no. 1, 1 janvier 2003 (2003-01-01), pages 52-61, XP019834046, ISSN: 1741-0444**
- **Jeffrey J. Goldberger: "Sympathovagal balance: how should we measure it?", American Journal of Physiology - Heart and Circulatory Physiology, 1 avril 1999 (1999-04-01), pages 1273-1280, XP055197528, UNITED STATES Extrait de l'Internet: URL:http://ajpheart.physiology.org/content /276/4/H1273**

**Description**

La présente invention concerne le domaine de l'analyse de la variabilité de la fréquence cardiaque d'un être humain ou d'un animal de compétition pour en déduire une information sur son état de forme ou de fatigue. Plus précisément, l'invention porte sur une méthode de détermination d'un indice représentatif de l'activité sympatho-vagale d'un être humain ou d'un animal de compétition. L'activité sympatho-vagale ou la balance sympatho-vagale désigne le ratio entre l'activité sympathique et l'activité parasympathique du système nerveux autonome de l'être humain ou du animal de compétition.

L'information déduite de l'analyse de la variabilité de la fréquence cardiaque reflète l'activité du système nerveux autonome, plus précisément l'influence des deux branches antagonistes de ce système que sont le système nerveux sympathique et le système nerveux parasympathique.

Cette information présente un intérêt dans le domaine de la médecine, notamment pour l'aide au diagnostic des maladies comprenant les troubles cardio-respiratoires, le diabète, le cancer, l'obésité, mais également dans le domaine de la détection du stress chez un individu ou encore dans le domaine du sport, que ce soit le sport de haut niveau pour les êtres humains ou le domaine du sport animal ou des animaux de compétitions. Le sport animal comprend notamment les courses d'animaux, par exemple les courses de chevaux, de lévriers, de chameaux ou dromadaires mais aussi d'autres disciplines telles que le dressage, notamment le dressage de chevaux, le saut d'obstacle, le parcours. Par la suite, on utilise le terme animal de compétition pour désigner les exemples d'animaux précités ainsi que tout autre type d'animal de course non mentionné explicitement, ou de façon générale tout animal utilisé dans une compétition sportive ou artistique.

Dans le cas du sport, un indicateur de l'activité sympatho-vagale peut être utilisé comme indicateur de l'état de forme d'un sportif et être utilisé pour améliorer et individualiser un entrainement d'un sportif, en particulier un sportif de haut niveau.

De la même façon, un tel indicateur peut être utilisé pour gérer l'entrainement d'un animal de compétition dans une discipline de sport animal.

L'état de l'art de l'analyse de la variabilité de la fréquence cardiaque d'un être humain contient principalement trois domaines d'analyse : le domaine temporel, le domaine non-linéaire et le domaine fréquentiel.

Les méthodes connues d'analyse dans le domaine temporel sont basées sur des indicateurs statistiques déterminés directement à partir de mesures consécutives de l'intervalle de temps entre deux battements cardiaques. Les documents [1], [2] et [3] décrivent de telles méthodes d'analyse dans le domaine temporel. Un inconvénient de ces méthodes est qu'elles ne permettent pas de fournir un indicateur qui traduit la part de l'activité sympathique et la part de l'activité parasympathique dans le fonctionnement du système nerveux autonome. Cette distinction présente une importance en particulier lorsqu'on souhaite caractériser l'état de forme ou de fatigue d'un sportif.

Les méthodes d'analyse non-linéaire proposent des indicateurs qui sont fortement corrélés à ceux déterminés par les méthodes dans le domaine temporel. La référence [7] décrit une méthode d'analyse non-linéaire. L'intérêt principal de ces méthodes réside dans la représentation graphique des indices sous la forme de diagramme de Poincaré, cependant elles présentent les mêmes inconvénients que les méthodes d'analyse dans le domaine temporel.

Les méthodes d'analyse dans le domaine fréquentiel sont les plus largement utilisées dans le domaine général de la santé car elles permettent de pallier la limitation précitée des méthodes temporelles en fournissant un indicateur qui donne une information à la fois sur l'activité sympathique et parasympathique du système nerveux autonome de l'individu. Les méthodes dans le domaine fréquentiel sont basées sur des statistiques dérivées de la mesure de la variabilité de la fréquence cardiaque. Les documents [1] et [4] décrivent de telles méthodes. Une méthode fréquentielle consiste à mesurer la densité spectrale de puissance d'une courbe de variabilité de fréquence cardiaque et à séparer l'énergie en deux bandes de fréquences, hautes et basses. Les basses fréquences donnent une information sur l'influence sympathique du système nerveux autonome tandis que les hautes fréquences donnent une information sur l'influence parasympathique du système nerveux autonome. Le ratio entre les deux bandes de fréquences fourni un indicateur de l'activité sympatho-vagal.

Cependant, les méthodes d'analyse dans le domaine fréquentiel présentent l'inconvénient d'être étroitement liées à la fréquence de respiration de l'individu. Ce lien a un impact fort pour le cas de sportifs car l'arythmie sinusale respiratoire peut grandement perturber ce type de marqueurs. En particulier, le document [5] montre les limitations des méthodes fréquentielles et le document [6] décrit plus précisément l'imprécision de ces mesures pour déterminer l'état de forme ou les phases de surentrainement chez les sportifs de haut niveau.

La présente invention vise à résoudre les limitations des solutions de l'art antérieur, en particulier des méthodes d'analyse dans le domaine temporel et fréquentiel, en proposant une méthode de détermination d'un indice représentatif de l'activité sympatho-vagal qui permet de caractériser à la fois l'influence sympathique et l'influence parasympathique du système nerveux autonome en restant indépendant de la fréquence de respiration de l'individu. L'invention vise à fournir une solution qui est adaptée à l'analyse de l'état de forme d'un sportif de haut niveau et qui peut être utilisée comme indicateur pour améliorer l'entrainement d'un sportif, en particulier pour détecter des phases de surentrainement. L'invention s'applique également à l'analyse de l'état de forme d'un animal de compétition tel qu'un cheval, un chien de course tel qu'un lévrier, un chameau ou un dromadaire.

L'invention permet également d'automatiser le traitement des mesures R-R pour supprimer les artefacts liés aux appareils de mesure.

L'invention propose une méthode mise en oeuvre par ordinateur qui peut être implémentée en tant qu'application logicielle dans un terminal portatif, par exemple un téléphone intelligent, ou dans un serveur sécurisé.

L'invention vise également à proposer une méthode d'élaboration d'une courbe de l'état de forme d'un sportif.

L'invention a ainsi pour objet une méthode, mise en oeuvre par ordinateur, de détermination d'un indice représentatif de l'activité sympatho-vagale d'un individu, ladite méthode comprenant les étapes suivantes :

- Recevoir une séquence comportant une pluralité de mesures, dites mesures R-R, de l'intervalle de temps entre deux battements cardiaques consécutifs de l'individu,
- Construire une première sous-séquence constituée des écarts positifs entre deux valeurs consécutives de la dite séquence reçue,
- Construire une seconde sous-séquence constituée des valeurs absolues des écarts négatifs entre deux valeurs consécutives de la dite séquence reçue,
- Calculer un indice égal à une valeur représentative de la comparaison entre le résultat d'une fonction statistique appliquée aux valeurs de la première sous-séquence et le résultat de ladite fonction statistique appliquée aux valeurs de la seconde sous-séquence.

Selon un aspect particulier de l'invention, l'indice calculé est égal au ratio entre une fonction statistique appliquée aux valeurs de la première sous-séquence et ladite fonction statistique appliquée aux valeurs de la seconde sous-séquence.

Selon un aspect particulier de l'invention, ladite fonction statistique est prise dans l'ensemble suivant : une moyenne, une variance, un écart type.

Selon une variante particulière de réalisation, la méthode selon l'invention comprend en outre une étape de filtrage de ladite séquence reçue consistant à supprimer des valeurs extrêmes et/ou des écarts extrêmes entre deux valeurs consécutives, résultant d'artefacts de mesure et correspondant à des valeurs physiologiquement impossibles.

Selon une variante particulière de réalisation, l'étape de filtrage comprend au moins la suppression des mesures R-R dont les valeurs sont supérieures à un premier seuil ou inférieures à un deuxième seuil, lesdits premier et deuxième seuils étant configurés en fonction de l'amplitude maximale et minimale physiologiquement possible de l'intervalle entre deux battements d'un cœur humain.

Selon une variante particulière de réalisation, l'étape de filtrage comprend au moins la suppression d'une mesure R-R si le rapport entre cette mesure R-R et la précédente est supérieur à un troisième seuil ou inférieur à un quatrième seuil, ledit troisième seuil et ledit quatrième seuil étant configurés en fonction des valeurs maximale et minimale physiologiquement possible dudit rapport.

Selon une variante particulière de réalisation, l'étape de filtrage comprend au moins la suppression d'une mesure R-R si :

- l'écart entre cette mesure et la précédente et l'écart entre cette mesure et la suivante est supérieur à un cinquième seuil, ou
- l'écart entre cette mesure et la précédente et l'écart entre cette mesure et la suivante est inférieur à un sixième seuil,
- ledit cinquième seuil et ledit sixième seuil étant configurés en fonction des valeurs maximale et minimale physiologiquement possible desdits écarts

Selon une variante particulière de réalisation, l'étape de filtrage comprend au moins la suppression d'une mesure R-R si :

- Le ratio entre cette mesure et la précédente et le ratio entre cette mesure et la suivante est supérieur à un septième seuil, ou
- Le ratio entre cette mesure et la précédente et le ratio entre cette mesure et la suivante est inférieur à un huitième seuil,
- ledit septième seuil et ledit huitième seuil étant configurés en fonction des valeurs maximale et minimale physiologiquement possible desdits ratios.

Selon une variante particulière de réalisation, l'étape de filtrage comprend au moins la suppression d'une mesure R-R si la valeur absolue de l'écart entre cette mesure et la précédente est supérieure à un neuvième seuil dépendant de l'écart type calculé sur l'ensemble des mesures de la séquence, ledit neuvième seuil étant configuré en fonction des valeurs maximale et minimale physiologiquement possible dudit écart.

Selon une variante particulière de réalisation, l'étape de filtrage comprend au moins la suppression d'une mesure R-R si :

- l'écart entre cette mesure et la précédente et l'écart entre cette mesure et la suivante sont supérieurs à un dixième seuil dépendant de l'écart type calculé sur l'ensemble des mesures de la séquence, ou
- l'écart entre cette mesure et la précédente et l'écart entre cette mesure et la suivante sont inférieurs à un onzième seuil dépendant de l'écart type calculé sur l'ensemble des mesures de la séquence,
- ledit dixième seuil et ledit onzième seuil étant configurés en fonction des valeurs maximale et minimale physiologiquement possible desdits écarts.

Selon une variante particulière de réalisation, la méthode selon l'invention comprend en outre une étape préalable à la construction de la première et de la seconde sous-séquences consistant à supprimer un nombre prédéterminé de mesures au début et à la fin de la séquence de sorte à corriger les effets de bords.

Selon un mode de réalisation particulier, l'invention s'applique également en remplaçant l'individu par un animal de compétition, par exemple un cheval, un chien de course, tel qu'un lévrier, ou un chameau ou un dromadaire.

L'invention a également pour objet un programme d'ordinateur comportant des instructions pour l'exécution de la méthode de détermination d'un indice représentatif de l'activité sympatho-vagale d'un individu selon l'invention, lorsque le programme est exécuté par un processeur ainsi qu'un support d'enregistrement lisible par un processeur sur lequel est enregistré un programme comportant des instructions pour l'exécution de la méthode de détermination d'un indice représentatif de l'activité sympatho-vagale d'un individu selon l'invention, lorsque le programme est exécuté par un processeur.

L'invention a encore pour objet un dispositif, par exemple terminal ou serveur, comprenant une mémoire et un processeur configurés pour mettre en oeuvre la méthode de détermination d'un indice représentatif de l'activité sympatho-vagale d'un individu selon l'invention.

L'invention a encore pour objet un système de détermination d'un indice représentatif de l'activité sympatho-vagale d'un individu comprenant un moyen de mesure d'une pluralité de mesures consécutives, dites mesures R-R, de l'intervalle de temps entre deux battements cardiaques de l'individu, un dispositif selon l'invention et un moyen de communication entre le moyen de mesure et ledit dispositif. Le moyen de mesure peut être un cardiofréquencemètre ou un textile instrumenté ou un électrocardiographe.

D'autres caractéristiques et avantages de la présente invention apparaîtront mieux à la lecture de la description qui suit en relation aux dessins annexés qui représentent :

- La figure 1, un organigramme détaillant les étapes de mise en œuvre de la méthode de détermination d'un indice représentatif de l'activité sympatho-vagale d'un individu,
- Les figures 2a et 2b deux diagrammes illustrant l'influence des étapes de filtrage appliquées à une séquence de mesures R-R afin de supprimer les artefacts de mesures,
- La figure 3, un schéma illustrant un système de mise en œuvre de l'invention,
- La figure 4a, un organigramme détaillant les étapes de mise en œuvre de la méthode d'élaboration d'une courbe d'évolution de l'état de forme d'un individu,
- La figure 4b, un schéma illustrant, sur un exemple, l'allure des séquences d'indice obtenues à chaque étape de la méthode de la figure 4a,
- La figure 5, un diagramme illustrant un exemple de courbe d'évolution de l'état de forme d'un individu obtenu à l'issue de la méthode décrite à l'appui de la figure 4a.

Un mode de réalisation de l'invention est tout d'abord décrit en détail pour son application à la surveillance de l'état de forme d'un être humain.

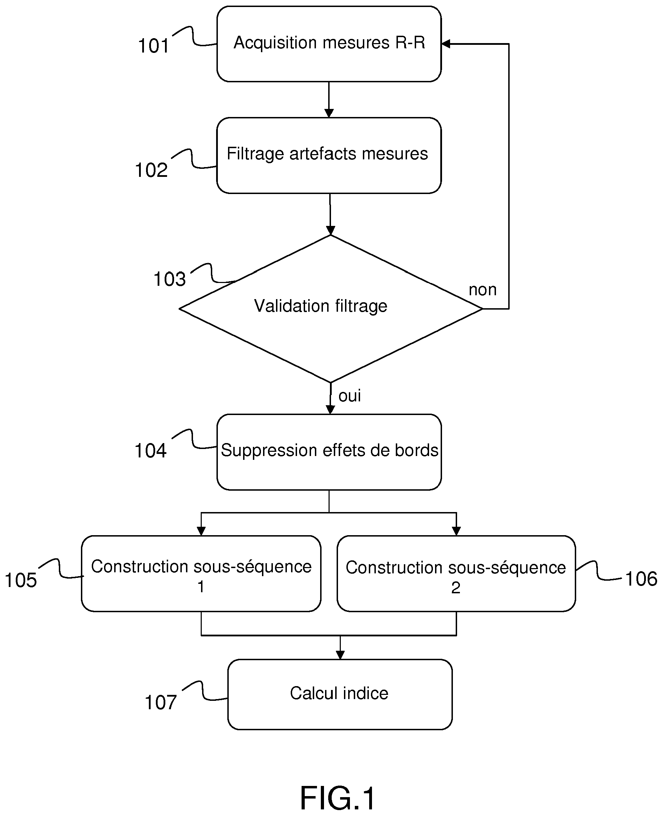

La figure 1 détaille, sur un organigramme, les étapes de mise en œuvre de la méthode, selon l'invention, de détermination d'un indice représentatif de l'activité sympatho-vagale d'un individu.

Selon une première étape 101, on collecte une pluralité de mesures de l'intervalle de temps entre deux battements cardiaques consécutifs d'un individu. Ces mesures sont réalisées entre deux pics d'amplitude consécutifs sur l'onde R d'un électrocardiogramme qui est l'onde de plus grande amplitude et sont désignées par l'expression « mesures R-R » bien connue du domaine. Les durées successives des intervalles R-R sont inversement proportionnelles aux fréquences cardiaques instantanées de l'individu.

L'acquisition de mesures R-R peut être effectuée à l'aide de différents capteurs. Elle peut se faire par mesure sur un électrocardiogramme enregistré à l'aide d'un cardiofréquencemètre ou par le biais de tout type de capteurs permettant la mesure d'un électrocardiogramme sur un individu ou directement la mesure des intervalles R-R. De tels capteurs comprennent en particulier une ceinture thoracique cardiaque, un textile instrumenté ou des capteurs déportés sur des zones du corps éloignées du cœur ou du thorax tel des capteurs positionnés sur le lobe de l'oreille ou sur l'extrémité d'un doigt. L'acquisition des mesures R-R peut être effectuée en milieu hospitalier par un personnel médical mais peut également, grâce à certains capteurs précités, être réalisée directement par l'individu. Le capteur de mesure peut notamment être embarqué dans un téléphone intelligent ou dans un bracelet montre intelligent.

L'acquisition des mesures R-R peut être réalisée en une ou plusieurs sessions d'acquisition. La durée totale de l'acquisition doit être suffisante pour permettre l'exécution de la méthode selon l'invention. En pratique, une durée d'acquisition au moins égale à cinq minutes est suffisante.

Les mesures R-R sont stockées dans une base de données sous la forme d'une séquence initiale. Préférentiellement, les mesures R-R sont acquises sur un individu dans une situation de repos.

Une deuxième étape 102 de la méthode selon l'invention permet le filtrage automatisé des artefacts de mesures dans la séquence initiale. Cette deuxième étape 102 produit en sortie une séquence de mesures R-R filtrées dans laquelle une partie des mesures sont éliminées car identifiées comme des mesures aberrantes résultant des imperfections des équipements de mesure.

Un avantage lié à cette deuxième étape 102 particulière est de permettre une automatisation du filtrage des artefacts alors que cette opération doit usuellement être réalisée par un opérateur et de façon manuelle.

Une façon de réaliser le filtrage de la séquence initiale est décrite à présent en se basant sur l'utilisation de six filtres consécutifs.

La séquence de mesures R-R initialement acquise est notée séquence L0. La séquence L0 comprend une pluralité de mesures R-R consécutives indicées par l'entier i qui varie de 0 à N-1, où N est le nombre de mesures réalisées.

Un premier filtre F1 est appliqué à la séquence initiale L0 afin de supprimer les valeurs extrêmes instantanées. Une mesure est supprimée de la séquence si sa valeur est supérieure à un premier seuil ou si sa valeur est inférieure à un deuxième seuil. Le premier et le deuxième seuil sont fixés en fonction de l'amplitude maximale et minimale envisageable pour l'intervalle entre deux battements cardiaques d'un cœur humain. Par exemple, le premier seuil peut être fixé à 5000 et le deuxième seuil à 200. Une plage de tolérance de plus ou moins 20% peut être envisagée autour de ces valeurs. Ces seuils sont configurables et peuvent notamment prendre en compte les caractéristiques personnelles de l'individu, notamment son profil de santé.

L'application du premier filtre F1 permet l'obtention d'une première séquence filtrée L1. Ce premier filtre F1 a pour avantage de permettre la suppression automatisée de valeurs physiologiquement impossibles dans la séquence de mesures R-R.

Un deuxième filtre F2 peut être appliqué à la première séquence filtrée L1 afin de supprimer les valeurs extrêmes mais cette fois de façon relative. Autrement dit, le deuxième filtre F2 vise à supprimer les mesures qui ont une valeur très supérieure ou très inférieure à la valeur de la mesure précédente. Une mesure est supprimée de la séquence L1 si le rapport entre la valeur de cette mesure et la valeur de la mesure précédente est supérieur à un troisième seuil ou inférieur à un quatrième seuil. Les seuils sont configurables de la même façon que pour le premier filtre F1. Une valeur possible pour le troisième seuil est 1,75. Une valeur possible pour le quatrième seuil est 0,25. Une plage de tolérance de plus ou moins 10% peut être envisagée autour de ces valeurs. L'application du deuxième filtre F2 permet l'obtention d'une deuxième séquence filtrée L2. Ce deuxième filtre F2 a pour avantage de permettre la suppression automatisée de valeurs physiologiquement impossibles dans la séquence de mesures R-R en prenant en compte les variations d'amplitude des mesures R-R spécifiques à chaque individu. Par exemple, les variations d'amplitude sont plus élevées chez un sportif de haut niveau que chez un individu sédentaire ou chez un patient hospitalisé. L'utilisation d'un filtrage relatif permet de s'adapter automatiquement au type de profil physiologique de l'individu.

Un troisième filtre F3 peut être appliqué à la deuxième séquence filtrée L2 afin de supprimer, de façon absolue, les écarts extrêmes entre deux mesures consécutives.

Une mesure est supprimée de la séquence L2 si

- l'écart entre cette mesure et la précédente et l'écart entre cette mesure et la suivante est supérieur à un cinquième seuil, ou
- l'écart entre cette mesure et la précédente et l'écart entre cette mesure et la suivante est inférieur à un sixième seuil.

Les seuils sont configurables de la même façon que pour les filtres précédents. Une valeur possible pour le cinquième seuil est 400. Une valeur possible pour le sixième seuil est -400. Une valeur possible pour le quatrième seuil est 0,25. Une plage de tolérance de plus ou moins 10% peut être envisagée autour de ces valeurs.

L'application du troisième filtre F3 permet l'obtention d'une troisième séquence filtrée L3. Le troisième filtre F3 a pour avantage de permettre la suppression automatisée de valeurs physiologiquement impossibles dans les écarts entre deux mesures R-R successives.

Un quatrième filtre F4 peut être appliqué à la troisième séquence filtrée L3 afin de supprimer, de façon relative, les écarts extrêmes entre deux mesures consécutives.

Une mesure est supprimée de la séquence L3 si

- le rapport entre cette mesure et la précédente et le ratio entre cette mesure et la suivante est supérieur à un septième seuil, ou
- le rapport entre cette mesure et la précédente et le ratio entre cette mesure et la suivante est inférieur à un huitième seuil.

Les seuils sont configurables de la même façon que pour les filtres précédents. Une valeur possible pour le septième seuil est 1,4. Une valeur possible pour le huitième seuil est 0,6. Une valeur possible pour le quatrième seuil est 0,25. Une plage de tolérance de plus ou moins 10% peut être envisagée autour de ces valeurs.

L'application du quatrième filtre F4 permet l'obtention d'une quatrième séquence filtrée L4. Le quatrième filtre F4 a pour avantage de permettre la suppression automatisée de valeurs physiologiquement impossibles dans les écarts entre deux mesures R-R successives en prenant en compte les variations d'amplitude desdits écarts spécifiques à chaque individu de façon similaire à l'avantage obtenu par le deuxième filtre F2 pour la suppression des valeurs physiologiquement impossibles.

Un cinquième filtre F5 peut être appliqué à la quatrième séquence filtrée L4 afin de supprimer des valeurs extrêmes par rapport à l'écart type des valeurs calculé sur l'ensemble de la séquence.

Une mesure est supprimée de la séquence L4 si la valeur absolue de l'écart entre cette mesure et la précédente est supérieure à un neuvième seuil dépendant de l'écart type calculé sur l'ensemble des mesures de la séquence. Le neuvième seuil est configurable et peut, par exemple, être pris égal à 4σ, où σ est égal à l'écart-type calculé sur l'ensemble des mesures de la séquence filtrée L4. Une plage de tolérance de plus ou moins 5% peut être envisagée autour de cette valeur.

L'application du cinquième filtre F5 permet l'obtention d'une quatrième séquence filtrée L5.

Un sixième filtre F6 peut être appliqué à la cinquième séquence filtrée L5 afin de supprimer des écarts extrêmes entre deux mesures consécutives, par rapport à l'écart type de l'ensemble des mesures.

Une mesure est supprimée de la séquence L6 si

- l'écart entre cette mesure et la précédente et l'écart entre cette mesure et la suivante sont supérieurs à un dixième seuil dépendant de l'écart type calculé sur l'ensemble des mesures de la séquence, ou
- l'écart entre cette mesure et la précédente et l'écart entre cette mesure et la suivante sont inférieurs à un onzième seuil dépendant de l'écart type calculé sur l'ensemble des mesures de la séquence.

Les seuils sont configurables de la même façon que pour les filtres précédents. Le dixième seuil est par exemple pris égal à 2,5σ et le onzième seuil est par exemple pris égal à -2,5σ. σ est égal à l'écart-type calculé sur l'ensemble des mesures de la séquence filtrée L5. Une plage de tolérance de plus ou moins 5% peut être envisagée autour de ces valeurs.

Le cinquième et le sixième filtre permettent d'affiner encore d'avantage le filtrage des artefacts de mesure. Les seuils de filtrage des quatre premiers filtres sont configurés de sorte à laisser passer une partie des artefacts de mesure d'amplitude faible mais de façon à ne pas filtrer ou à limiter le filtrage de mesures contenant une information réelle.

L'application du cinquième filtre et du sixième filtre permet de supprimer le bruit résiduel restant après l'application des quatre premiers filtres.

L'application successive des six filtres décrit ci-dessus a pour avantage un filtrage progressif du bruit de mesure et permet de minimiser la probabilité de filtrage de mesures contenant de l'information.

Le choix des seuils de filtrage peut avantageusement être choisi dans les plages de tolérance indiquées pour chaque filtre. Les plages de tolérance autour de la valeur numérique préférentielle indiquée pour chaque seuil sont choisies de sorte à être plus larges pour les premiers filtres et plus étroites pour les derniers filtres. Ce choix provient du fait que l'application successive des six filtres a pour effet de filtrer progressivement les artefacts liés aux erreurs de mesure en limitant au maximum la probabilité de filtrage d'une information.

Sans sortir du cadre de l'invention, les étapes de filtrage décrites ci-dessus peuvent être réalisées par des filtres différents pour obtenir le même résultat. Par exemple, les six étapes de filtrage peuvent être réalisées à l'aide d'un filtre unique configuré pour appliquer directement un ou plusieurs critères de filtrage des mesures ou des écarts en fonction de leur valeur.

L'application successive des six filtres décrits ci-dessus dans l'ordre indiqué présente des avantages particuliers qui ont été explicités. Cependant, l'invention n'est pas limitée à cette forme particulière de réalisation du filtrage. En particulier, l'invention peut être réalisée en appliquant seulement une partie des filtres, par exemple uniquement le premier filtre ou uniquement les N premiers filtres avec N un nombre entier strictement inférieur à 6.

La fonction de l'étape de filtrage 102 est de supprimer les artefacts de mesure éventuels qui peuvent apparaitre du fait de l'imperfection des capteurs utilisés. L'étape de filtrage 102 prend en compte la connaissance de l'évolution des battements cardiaques chez l'être humain et en particulier les valeurs extrêmes possibles pour les mesures R-R.

Si les capteurs de mesures R-R utilisés permettent de fournir des mesures sans, ou avec un nombre limité, d'artefacts, il est également envisageable que l'étape de filtrage 102 soit optionnelle.

A l'issu de l'ensemble des étapes de filtrage, on obtient une séquence filtrée finale.

L'ensemble des seuils de filtrage peuvent être configurés conjointement ou indépendamment les uns des autres en fonction de caractéristiques présupposées de l'évolution du battement cardiaque d'un être humain. Les seuils de filtrage sont adaptatifs et sont notamment configurés pour minimiser la probabilité de filtrer une mesure contenant de l'information. Les seuils peuvent être également configurés en prenant en compte des caractéristiques personnelles de l'individu, par exemple le fait que l'individu soit un sportif de haut niveau.

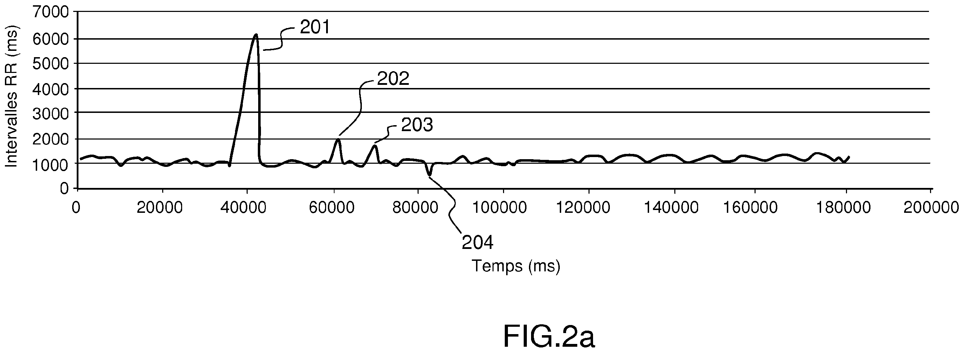

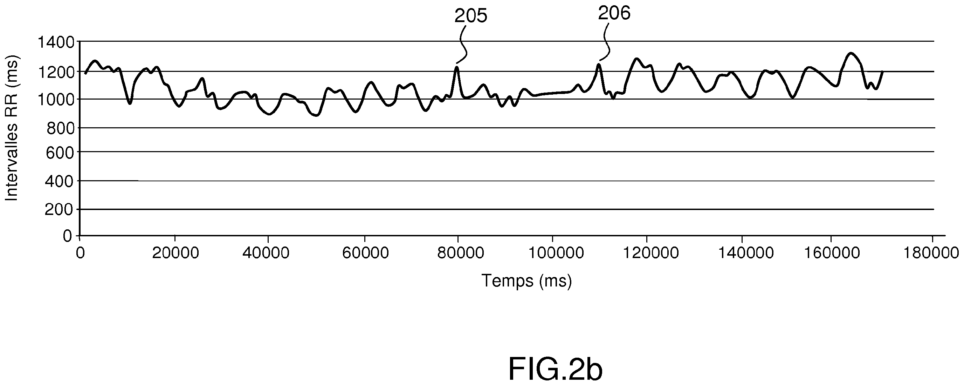

Les figures 2a et 2b illustrent, sur deux diagrammes, le profil des mesures R-R sur la durée d'une séquence à deux stades du procédé.

La figure 2a représente un exemple de séquence initiale L0 de mesures R-R acquises pour un individu donné. Les références 201,202,203,204 identifient sur la courbe de mesure différents artefacts de mesure.

La mesure 201 correspond à une mesure dont la valeur est très élevée et peut être filtrée par l'application du premier filtre F1.

La mesure 202 présente une valeur élevée relativement à la mesure précédente et peut être filtrée par l'application du deuxième filtre F2.

La mesure 203 présente un écart absolu important par rapport à la mesure précédente et à la mesure suivante et peut être filtrée par l'application du troisième filtre F3.

La mesure 204 présente un écart relatif important par rapport à la mesure précédente et à la mesure suivante et peut être filtrée par l'application du quatrième filtre F4.

La figure 2b représente la séquence filtrée obtenue à l'issue de l'application successive des quatre premiers filtres F1,F2,F3,F4.

Les artefacts les plus significatifs ont été supprimés, cependant certaines mesures aberrantes peuvent encore être effacées. La mesure 205 correspond à une valeur extrême par rapport à l'écart type de l'ensemble des mesures et peut être supprimé par application du cinquième filtre F5. La mesure 206 correspond à un écart extrême par rapport à l'écart type de l'ensemble des mesures et peut être supprimé par application du sixième filtre F6.

Une troisième étape 103 de validation du filtrage est ensuite opérée afin de vérifier que la séquence filtrée obtenue contient suffisamment de mesures pour pouvoir calculer un indicateur fiable. La troisième étape 103 consiste à tester que le nombre de mesures de la séquence filtrée finale est au moins égal à un nombre minimum de mesures ou à un pourcentage du nombre de mesures présentes dans la séquence initiale L0. Par exemple, le test selon la troisième étape 103 peut consister à vérifier que la séquence filtrée contient au moins 90% des éléments de la séquence initiale L0.

Si le nombre de mesures contenues dans la séquence filtrée finale est jugé trop faible, la phase d'acquisition de la méthode est inexploitable et on effectue alors une nouvelle étape 101 d'acquisition de mesures R-R.

Selon une quatrième étape 104 de la méthode, les effets de bords affectant les mesures R-R acquises sont corrigés.

Lors d'une séance d'acquisition de mesures, le début de la séquence acquise peut être perturbé car il faut un temps minimum pour obtenir une stationnarité dans les mesures cardiaques réalisées. De même, la fin de la séquence acquise peut également être perturbée si, par exemple, l'individu se relève ou effectue des gestes brusques. Les effets de bord peuvent induire des dérives linéaires progressives qui ne sont pas détectées par l'étape de filtrage 102 qui vise principalement à éliminer des ruptures de continuité brutales.

L'étape 104 consiste à supprimer un nombre de mesures prédéterminé au début et à la fin de la séquence. Ce nombre peut correspondre à une durée prédéterminée, par exemple une durée égale à 30 secondes. La durée de la portion supprimée au début de la séquence correspond au temps nécessaire pour arriver à un état stationnaire. La durée de la portion supprimée à la fin de la séquence correspond au temps s'écoulant entre la fin de l'état stationnaire, qui peut être initiée par un mouvement de l'individu, et la fin de l'enregistrement.

Optionnellement, l'étape 104 peut être éliminée si le capteur de mesure utilisé permet d'obtenir directement une séquence de mesures correspondant totalement à un état stationnaire du rythme cardiaque, par exemple, si le début et la fin de l'enregistrement peuvent être automatisés sans intervention de l'individu.

A partir de la séquence de mesures R-R filtrée obtenue à l'issue des étapes précédentes, on construit ensuite, dans une cinquième étape 105, une sous-séquence composée des écarts positifs entre deux valeurs consécutives de la séquence initiale. Autrement dit, si on note $\{RR_0, ... RR_i, ..., RR_{N-1}\}$ les mesures de la séquence obtenue en sortie de l'étape 104, et $\{k_0 = RR_1 - RR_0 ; k_1 = RR_2 - RR_1 ; ... ; k_n = RR_{n+1} - RR_n\}$ les écarts positifs entre deux valeurs consécutives, on construit une sous-séquence composée des valeurs $k_i$ positives.

Dans une sixième étape 106, on construit également une deuxième sous-séquence composée cette fois des valeurs absolue des écarts négatifs entre deux valeurs consécutives. Cette deuxième sous-séquence comprend les valeurs absolues des valeurs $k_i$ négatives.

Enfin, dans une dernière étape 107, on détermine l'indice représentatif de l'activité sympatho-vagale de l'individu à partir des valeurs des deux sous-séquences déterminées aux étapes 105 et 106. Plus précisément, l'indice déterminé à l'étape 107 est calculé comme une information représentative de la comparaison entre une première valeur statistique représentative des valeurs de la première sous-séquence et une seconde valeur statistique représentative des valeurs de la seconde sous-séquence.

Par exemple, l'indice peut être calculé comme le ratio entre la moyenne des valeurs de la seconde sous-séquence et la moyenne des valeurs de la première sous-séquence :

$$I=moy(SS2)/moy(SS1) \quad (1)$$

L'indice peut également être calculé en représentation logarithmique :
$I=In(moy(SS2)/moy(SS1))*10$, où In désigne la fonction logarithme népérien. Mais l'indice peut également être égal au ratio entre l'écart-type des valeurs de la seconde sous-séquence et l'écart type des valeurs de la première sous-séquence :

$$I=\text{écart-type}(SS2)/\text{écart-type}(SS1) \quad (2)$$

L'objectif du calcul de l'indice selon l'invention est d'obtenir une information représentative de la comparaison entre les valeurs de la première sous-séquence (écarts positifs) qui représentent la proportion de l'influence du système nerveux parasympathique et les valeurs de la seconde sous-séquence (valeur absolue des écarts négatifs) qui représentent la proportion de l'influence du système nerveux sympathique. Par exemple, si l'indice est donné par la formule (1) ou la formule (2), une valeur élevée de l'indice correspond à une prépondérance du système sympathique tandis qu'une valeur faible correspond à une prépondérance du système parasympathique. Une valeur de l'indice proche de la valeur 1 correspond à une situation équilibrée entre l'influence des deux systèmes.

De façon générale, l'indice calculé à l'étape 107 peut être formulé comme le ratio entre une fonction de la première sous-séquence et une fonction de la seconde sous-séquence. Bien entendu, le ratio inverse peut également être utilisé comme indice.

L'indice ainsi calculé par la méthode selon l'invention donne une information fiable sur l'état de forme de l'individu. En particulier, dans le cas d'un sportif de haut niveau, l'indice selon l'invention permet, à partir d'une méthode d'analyse temporelle, de caractériser à la fois l'influence de l'activité sympathique et l'influence de l'activité parasympathique sans être dépendante de la respiration de l'individu.

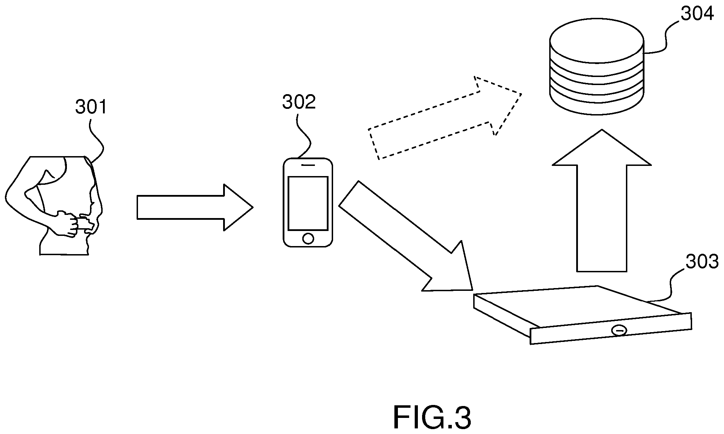

La figure 3 schématise un exemple de système pour la mise en œuvre de la méthode selon l'invention.

L'acquisition des mesures R-R est réalisée à l'aide d'un capteur 301 de mesure qui peut être un cardiofréquencemètre ou un capteur parmi ceux déjà décrits : textile instrumenté ou autre capteur embarqué.

Les mesures R-R enregistrées peuvent être transmises du capteur 301 vers un téléphone intelligent 302 ou un terminal du type tablette électronique ou ordinateur portatif ou tout autre dispositif électronique apte à communiquer avec le capteur de mesure 301.

La séquence enregistrée par le terminal 302 peut ensuite être envoyée vers un serveur sécurisé 303 si l'on ne souhaite pas que les données acquises soient accessibles de façon non sécurisée. Le serveur sécurisé 303 peut être associé à une base de données 304 pour stocker les différentes données nécessaires à l'exécution de la méthode selon l'invention.

La méthode selon l'invention peut être implémentée à partir d'éléments matériel et/ou logiciel. Elle peut notamment être mise en œuvre en tant que programme d'ordinateur comportant des instructions pour son exécution. Le programme d'ordinateur peut être enregistré sur un support d'enregistrement lisible par un processeur. Le support peut être électronique, magnétique, optique ou électromagnétique. Le programme d'ordinateur peut être exécuté par le serveur sécurisé 303. Le serveur sécurisé peut utiliser, pour mettre en œuvre l'invention, un ou plusieurs circuits électronique dédiés ou un circuit à usage général. La technique de l'invention peut se réaliser sur une machine de calcul reprogrammable (un processeur ou un micro contrôleur par exemple) exécutant un programme comprenant une séquence d'instructions, ou sur une machine de calcul dédiée (par exemple un ensemble de portes logiques comme un FPGA ou un ASIC, ou tout autre module matériel).

A titre d'exemple d'architecture matérielle adaptée à mettre en œuvre l'invention, le serveur sécurisé 303 peut comporter un bus de communication auquel sont reliés une unité centrale de traitement ou microprocesseur (CPU, acronyme de « *Central Processing Unit* » en anglais; une mémoire morte (ROM, acronyme de « *Read Only Memory* » en anglais) pouvant comporter les programmes nécessaires à la mise en œuvre de l'invention; une mémoire vive ou mémoire cache (RAM, acronyme de « *Random Access Memory* » en anglais) comportant des registres adaptés à enregistrer des variables et paramètres créés et modifiés au cours de l'exécution des programmes précités ; et une interface de communication ou E/S (I/O acronyme de « *Input/ouput* » en anglais) adaptée à transmettre et à recevoir des données.

Le code logiciel selon l'invention peut être exécuté sur n'importe quel processeur approprié (par exemple, un microprocesseur) ou cœur de processeur ou un ensemble de processeurs, qu'ils soient prévus dans un dispositif de calcul unique ou répartis entre plusieurs dispositifs de calcul (par exemple tels qu'éventuellement accessibles dans l'environnement du dispositif). Le code exécutable de chaque programme permettant au dispositif programmable de mettre en œuvre les processus selon l'invention, peut être stocké, par exemple, dans le disque dur ou en mémoire morte. De manière générale, le ou les programmes pourront être chargés dans un des moyens de stockage du dispositif avant d'être exécutés. L'unité centrale peut commander et diriger l'exécution des instructions ou portions de code logiciel du ou des programmes selon l'invention, instructions qui sont stockées dans le disque dur ou dans la mémoire morte ou bien dans les autres éléments de stockage précités.

Si la sécurisation des données est moins cruciale, la méthode selon l'invention peut également être implémentée directement dans le terminal 302 qui comprend alors une base de données 304 ou une mémoire permettant le stockage des séquences de mesures R-R et des indices de forme générés par l'invention.

Le programme d'ordinateur selon l'invention peut être stocké dans ou sur un médium de stockage amovible (par exemple une carte SD, un DVD ou Bluray, un moyen de stockage de masse tel qu'un disque dur e.g. un SSD) ou non-amovible, volatile ou non-volatile, ce médium de stockage étant lisible partiellement ou totalement par un ordinateur ou un processeur. Le support lisible par ordinateur peut être transportable ou communicable ou mobile ou transmissible (i.e. par un réseau de télécommunication 2G, 3G, 4G, Wifi, fibre optique ou autre).

La référence à un programme d'ordinateur qui, lorsqu'il est exécuté, effectue l'une quelconque des fonctions décrites précédemment, ne se limite pas à un programme d'application s'exécutant sur un ordinateur hôte unique. Au contraire, les termes programme d'ordinateur et logiciel sont utilisés ici dans un sens général pour faire référence à tout type de code informatique (par exemple, un logiciel d'application, un micro logiciel, un microcode, ou toute autre forme d'instruction d'ordinateur) qui peut être utilisé pour programmer un ou plusieurs processeurs pour mettre en œuvre des aspects des techniques décrites ici. Les moyens ou ressources informatiques peuvent notamment être distribués ("*Cloud computing*"), éventuellement avec selon des technologies de pair-à-pair.

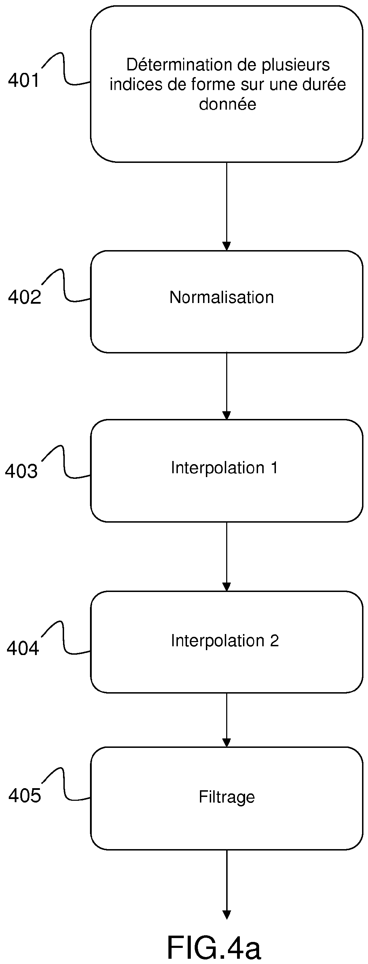

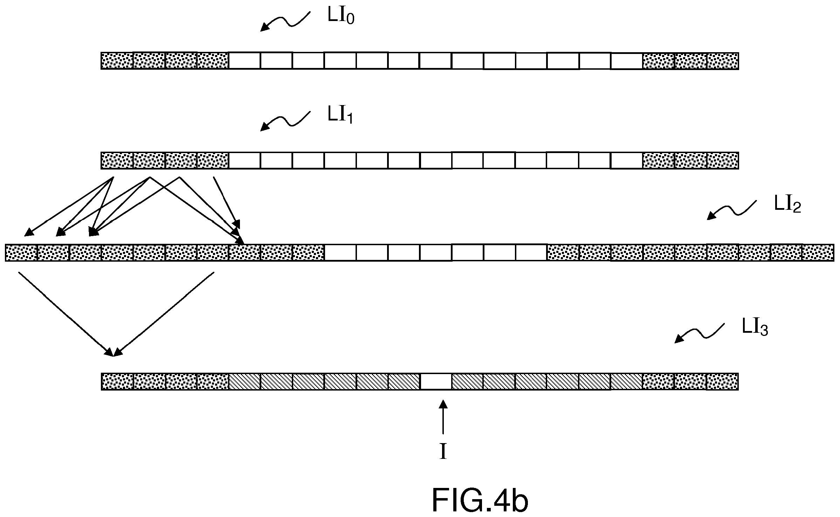

On décrit à présent une méthode d'élaboration d'une courbe de l'état de forme d'un individu à partir de l'indice représentatif de l'activité sympatho-vagale déterminé selon l'invention. L'organigramme de cette méthode est schématisé aux figures 4a et 4b. Par la suite, on appelle indice de forme, un indice généré par la méthode selon l'invention décrite précédemment.

Selon une première étape 401, on calcule une pluralité d'indices de forme sur une durée donnée pour le même individu. Les indices sont calculés à une période fixe, par exemple une période égale à un jour ou plusieurs jours. Cependant, à certaines occurrences de la période, les calculs d'indices peuvent être omis. Le nombre d'indices de forme calculés dépend notamment du nombre d'acquisitions de mesures R-R effectuées par l'individu. Par exemple, l'individu peut effectuer une séance d'acquisition par jour mais peut omettre cette séance certains jours.

A l'issue de la première étape 401, on obtient une première liste d'indices $LI_0$. Sur la figure 4b, la liste $LI_0$ est représentée par une succession de cases. Les cases grisées correspondent aux périodes où un indice a effectivement été calculé tandis que les cases blanches correspondent à des périodes où aucun indice n'a été calculé. Dans l'exemple de la figure 4b, on considère une période d'acquisition égale à un jour et une séquence de durée totale égale à 20 jours. Selon cet exemple, une période continue de 13 jours intervient pendant laquelle aucune acquisition de mesures R-R et aucun calcul d'indice n'a été effectué. Cet exemple est donné à titre purement illustratif et nullement limitatif. Un autre scénario consisterait à effectuer un calcul d'indice tous les jours pendant les 20 jours.

Cette première liste est ensuite normalisée 402. La normalisation consiste à calculer la moyenne M des valeurs non nulles de la première liste $LI_0$ et son écart type $\sigma$ puis à effectuer l'opération de normalisation suivante :

$$I_{norm} = (I-M)/ \sigma$$

Pour effectuer l'opération de normalisation, les cases de la première liste $LI_0$ qui présentent des valeurs nulles ne sont pas prises en compte.

On obtient une deuxième liste normalisée $LI_1$ qui comporte autant d'éléments que la première liste $LI_0$. L'opération de normalisation permet d'obtenir des valeurs d'indice de forme qui peuvent être comparées sur différentes périodes de temps et qui prennent en compte les critères physiques de l'individu.

Dans une troisième étape 403, on effectue une première interpolation des éléments de la liste normalisée $LI_1$. L'interpolation présente un paramètre P égal au nombre de valeurs sur laquelle l'interpolation est effectuée. Le paramètre P est configuré en prenant en compte les caractéristiques du profil de l'individu dont on souhaite évaluer la courbe de forme. Par expérimentation, le paramètre P est pris égal à 7 jours pour le cas d'un sportif de haut niveau. Le paramètre P est préférentiellement un nombre entier impair.

Pour construire la liste interpolée $LI_2$, on ajoute tout d'abord (P-1)/2 éléments en début et en fin de liste. Chaque élément de la liste interpolée est calculé comme la moyenne glissante de P éléments de la liste normalisée $LI_1$. La fenêtre de la moyenne glissante est toujours de durée égale à P éléments, mais les éléments nuls compris dans cette fenêtre ne sont pas pris en compte dans le calcul de la moyenne. Ainsi, il est possible de calculer un élément de la liste interpolée $LI_2$ si il existe au moins un élément non nul dans la fenêtre glissante de durée P appliquée à la liste normalisée $LI_1$. L'opération d'interpolation 402 permet de lisser les valeurs afin de supprimer les périodes sans indice, si ces périodes sont inférieures à la taille P de la fenêtre glissante.

La liste interpolée $LI_2$ obtenue est représentée sur la figure 4b, elle contient P-1 éléments supplémentaires par rapport à la liste normalisée $LI_1$. Comme la liste normalisée $LI_1$ comporte une période de durée supérieure à P (pris égal à 7 dans l'exemple de la figure 4b) pendant laquelle aucun indice n'est calculé, il en résulte que la liste interpolée $LI_2$ comporte encore plusieurs cases sans indice.

Pour résoudre ce problème et tenter d'obtenir une courbe continue, dans une variante de réalisation de l'invention, on opère 404 une seconde interpolation de même paramètre P que la première itération. Cette seconde interpolation permet également d'obtenir une liste finale $LI_3$ qui comporte le même nombre d'éléments que la liste initiale $LI_0$.

Lorsque le nombre d'éléments consécutifs sans indice, dans la liste interpolée $LI_2$, est supérieur ou égal à P (ce qui est le cas dans l'exemple de la figure 4b où P est égal à 7), on opère une étape supplémentaire de filtrage 405.

Cette étape de filtrage 405 consiste à supprimer les P-1 éléments situés avant un élément nul ainsi que les P-1 éléments situés après un élément nul. En effet, lorsque des éléments nuls subsistent après la seconde interpolation 404, cela signifie qu'un trop grand nombre d'éléments nuls consécutifs étaient présent dans la liste initiale et que par conséquent les valeurs interpolées dans cette période ne sont pas fiables. On préfère donc supprimer les valeurs calculées et conserver une période sans indices dans la liste finale au lieu d'élaborer des valeurs interpolées non fiables. Sur la figure 4b, la liste $LI_3$ contient encore un élément nul I après la seconde interpolation 404. Les 6 éléments situés avant l'élément I ainsi que les 6 éléments situés après (représentés en hachures) sont remis à zéro dans la liste finale.

L'étape de filtrage 405 permet donc de gérer les scénarii où aucun indice n'a été calculé pendant une longue période et où l'utilisateur souhaite, après cette période, à nouveau évaluer son indice de forme.

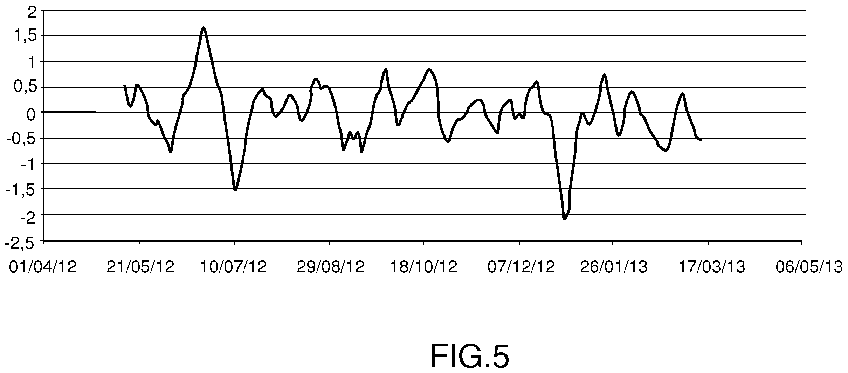

La figure 5 représente l'allure de la courbe d'évolution de l'état de forme d'un individu obtenue par application de la méthode décrite ci-dessus.

La période d'évaluation commence le 21/05/2012 et s'achève le 17/03/2013. Les valeurs de la courbe sont centrées autour de zéro. Les valeurs positives indiquent une évolution vers un état de forme tandis que les valeurs négatives indiquent une évolution vers un état de fatigue.

La génération de cette courbe de l'évolution de l'état de forme est notamment avantageuse dans le cas du suivi de l'entrainement d'un sportif de haut niveau. Le sportif peut, à n'importe quel moment, requérir l'établissement de sa courbe d'évolution d'état de forme même après une période longue sans requête. La normalisation des valeurs d'indice permet d'obtenir des valeurs cohérentes et comparables entre elles, même sur une longue durée et qui prennent en compte les caractéristiques physiques spécifiques de l'individu. Une génération de cette courbe apporte également de nombreux avantages dans le cadre d'un suivi médical (évolution d'une pathologie, réhabilitation,...) avec une mesure régulière de l'état de forme du patient.

La méthode d'élaboration d'une courbe de l'état de forme peut être implémentée à partir d'éléments matériel et/ou logiciel. Elle peut notamment être mise en œuvre en tant que programme d'ordinateur comportant des instructions pour son exécution. Le programme d'ordinateur peut être enregistré sur un support d'enregistrement lisible par un processeur. Il peut être exécuté par le serveur sécurisé 303 ou directement sur un terminal 302. La courbe peut être générée sur requête d'un utilisateur. La requête peut être transmise au serveur sécurisé 303 via le terminal 302 qui peut être un téléphone intelligent, une tablette numérique, un ordinateur portable ou tout autre équipement électronique apte à communiquer sans fils avec un serveur à distance. La courbe générée peut être transmise par le serveur 303 vers le terminal 302 puis affichée sur l'écran du terminal 302.

La courbe de l'état de forme générée selon l'invention présente l'avantage de pouvoir fournir une information fiable et rapide sur l'état de forme d'un individu. Elle présente notamment un intérêt pour un entraineur d'un sportif de haut niveau qui peut utiliser cette courbe afin d'améliorer l'entrainement du sportif.

Des statistiques peuvent être élaborées à partir de la courbe de l'état de forme, en particulier, la valeur la plus élevée et la plus faible sur une période donnée ou encore la moyenne la plus élevée et la plus faible sur une sous-période, par exemple égale à une semaine, la sous-période pouvant correspondre à un cycle d'entrainement.

Des seuils de détection d'événements anormaux peuvent également élaborés, par exemple un seuil de valeur d'indice de forme en dessous duquel l'état de fatigue du sportif est tel qu'il y a risque de blessures.

Sans sortir du cadre de l'invention, celle-ci s'applique également à la surveillance de l'état de forme d'un animal de compétition, notamment dans le cadre de disciplines sportives animales. L'animal de compétition peut être un animal de course, par exemple un cheval de course, un chien de course ou un chameau ou dromadaire de course. L'invention peut également s'étendre à d'autres disciplines sportives animales ou compétitions animales, notamment équestres, tels que le dressage, le saut d'obstacles ou le parcours, pour les chevaux.

La méthode de détermination d'un indice représentatif de l'activité sympatho-vagale d'un individu telle que décrite à la figure 1 peut s'appliquer à l'identique pour un animal de compétition. Les différents seuils utilisés dans les différentes étapes de la méthode décrite peuvent être adaptés ou réévalués en fonction du niveau d'entrainement de l'animal.

**Références**

[1] "Heart rate variability. Standards of measurement, physiological interprétation and clinical use", American Heart Association Inc.: European Society of Cardiology, 1996.

[2] "The impact of breathing on HRV measurements: Implications for the longitudinal follow-up of athletes", Damien Saboul et al, European Journal of Sport Science, 2013.

[3] "Training adaptation and heart rate variability in elite endurance athletes: opening the door to effective monitoring", Daniel J. Plews, Springer International Publishing Switzerland 2013.

[4] "Conversion from vagal to sympathetic prédominance with strenuous training in high performance world class athletes", Ferdinando Iellamo, American Heart Association, 2002

[5] "Sympathovagal Balance, a critical appraisal", Dwain L. Eckberg, American heart association, inc. 1997.

[6] " The breathing effect of the LF/HF ratio in the heart rate variability measurements of athletes", Damien Saboul et al., European Journal of sport science, 2012.

[7] " Correlations between the Poincaré Plot and conventional heart rate variability parameters assessed during paced breathing", Guzik et al, J.Physiol. Sci. vol 57, n°1, feb 2007, pp 63-71.

**Revendications**

1. Méthode, mise en œuvre par ordinateur, de détermination d'un indice représentatif de l'activité sympatho-vagale d'un individu ou d'un animal de compétition, ladite méthode comprenant les étapes suivantes :

   - Recevoir (101) une séquence comportant une pluralité de mesures, dites mesures R-R, de l'intervalle de temps entre deux battements cardiaques consécutifs de l'individu ou de l'animal de compétition, la méthode étant **caractérisée en ce qu'**elle comprend en outre:

- Construire (105) une première sous-séquence constituée des écarts positifs entre deux valeurs consécutives de la dite séquence reçue,
- Construire (106) une seconde sous-séquence constituée des valeurs absolues des écarts négatifs entre deux valeurs consécutives de la dite séquence reçue,
- Calculer (107) un indice égal à une valeur représentative de la comparaison entre le résultat d'une fonction statistique appliquée aux valeurs de la première sous-séquence et le résultat de ladite fonction statistique appliquée aux valeurs de la seconde sous-séquence.

2. Méthode de détermination d'un indice représentatif de l'activité sympatho-vagale d'un individu ou d'un animal de compétition selon la revendication 1 dans laquelle l'indice calculé est égal au ratio entre une fonction statistique appliquée aux valeurs de la première sous-séquence et ladite fonction statistique appliquée aux valeurs de la seconde sous-séquence.

3. Méthode de détermination d'un indice représentatif de l'activité sympatho-vagale d'un individu ou d'un animal de compétition selon la revendication 2 dans laquelle ladite fonction statistique est prise dans l'ensemble suivant : une moyenne, une variance, un écart type.

4. Méthode de détermination d'un indice représentatif de l'activité sympatho-vagale d'un individu ou d'un animal de compétition selon l'une des revendications précédentes comprenant en outre une étape de filtrage (102) de ladite séquence reçue consistant à supprimer des valeurs extrêmes et/ou des écarts extrêmes entre deux valeurs consécutives, résultant d'artefacts de mesure et correspondant à des valeurs physiologiquement impossibles.

5. Méthode de détermination d'un indice représentatif de l'activité sympatho-vagale d'un individu ou d'un animal de compétition selon la revendication 4 dans laquelle l'étape de filtrage (102) comprend au moins la suppression des mesures R-R dont les valeurs sont supérieures à un premier seuil ou inférieures à un deuxième seuil, lesdits premier et deuxième seuils étant configurés en fonction de l'amplitude maximale et minimale physiologiquement possible de l'intervalle entre deux battements d'un cœur humain ou d'un cœur d'un animal de compétition.

6. Méthode de détermination d'un indice représentatif de l'activité sympatho-vagale d'un individu ou d'un animal de compétition selon l'une des revendications 4 ou 5 dans laquelle l'étape de filtrage (102) comprend au moins la suppression d'une mesure R-R si le rapport entre cette mesure R-R et la précédente est supérieur à un troisième seuil ou inférieur à un quatrième seuil, ledit troisième seuil et ledit quatrième seuil étant configurés en fonction des valeurs maximale et minimale physiologiquement possible dudit rapport

7. Méthode de détermination d'un indice représentatif de l'activité sympatho-vagale d'un individu ou d'un animal de compétition selon l'une des revendications 4 à 6 dans laquelle l'étape de filtrage (102) comprend au moins la suppression d'une mesure R-R si :

- l'écart entre cette mesure et la précédente et l'écart entre cette mesure et la suivante est supérieur à un cinquième seuil, ou
- l'écart entre cette mesure et la précédente et l'écart entre cette mesure et la suivante est inférieur à un sixième seuil,
- ledit cinquième seuil et ledit sixième seuil étant configurés en fonction des valeurs maximale et minimale physiologiquement possible desdits écarts

8. Méthode de détermination d'un indice représentatif de l'activité sympatho-vagale d'un individu ou d'un animal de compétition selon l'une des revendications 4 à 7 dans laquelle l'étape de filtrage (102) comprend au moins la suppression d'une mesure R-R si :

- Le ratio entre cette mesure et la précédente et le ratio entre cette mesure et la suivante est supérieur à un septième seuil, ou
- Le ratio entre cette mesure et la précédente et le ratio entre cette mesure et la suivante est inférieur à un huitième seuil,
- ledit septième seuil et ledit huitième seuil étant configurés en fonction des valeurs maximale et minimale physiologiquement possible desdits ratios.

9. Méthode de détermination d'un indice représentatif de l'activité sympatho-vagale d'un individu ou d'un animal de compétition selon l'une des revendications 4 à 8 dans laquelle l'étape de filtrage (102) comprend au moins la suppression d'une mesure R-R si la valeur absolue de l'écart entre cette mesure et la précédente est supérieure à un neuvième seuil dépendant de l'écart type calculé sur l'ensemble des mesures de la séquence, ledit neuvième seuil étant configuré en fonction des valeurs maximale et minimale physiologiquement possible dudit écart.

10. Méthode de détermination d'un indice représentatif de l'activité sympatho-vagale d'un individu ou d'un animal de compétition selon l'une des revendications 4 à 9 dans laquelle l'étape de filtrage (102) comprend au moins la suppression d'une mesure R-R si :

- l'écart entre cette mesure et la précédente et l'écart entre cette mesure et la suivante sont supérieurs à un dixième seuil dépendant de l'écart type calculé sur l'ensemble des mesures de la séquence, ou
- l'écart entre cette mesure et la précédente et l'écart entre cette mesure et la suivante sont inférieurs à un onzième seuil dépendant de l'écart type calculé sur l'ensemble des mesures de la séquence,
- ledit dixième seuil et ledit onzième seuil étant configurés en fonction des valeurs maximale et minimale physiologiquement possible desdits écarts.

11. Méthode de détermination d'un indice représentatif de l'activité sympatho-vagale d'un individu ou d'un animal de compétition selon l'une des revendications précédentes comprenant en outre une étape (104) préalable à la construction de la première et de la seconde sous-séquences consistant à supprimer un nombre prédéterminé de mesures au début et à la fin de la séquence de sorte à corriger les effets de bords.

12. Méthode de détermination d'un indice représentatif de l'activité sympatho-vagale d'un individu ou d'un animal de compétition selon l'une des revendications précédentes dans laquelle l'animal de compétition est un cheval.

13. Programme d'ordinateur comportant des instructions pour l'exécution de la méthode de détermination d'un indice représentatif de l'activité sympatho-vagale d'un individu ou d'un animal de compétition selon l'une quelconque des revendications 1 à 12, lorsque le programme est exécuté par un processeur.

14. Support d'enregistrement lisible par un processeur sur lequel est enregistré un programme d'ordinateur selon la revendication 13.

15. Dispositif comprenant une mémoire et un processeur configurés pour mettre en œuvre la méthode de détermination d'un indice représentatif de l'activité sympatho-vagale d'un individu ou d'un animal de compétition selon l'une quelconque des revendications 1 à 12.

16. Système de détermination d'un indice représentatif de l'activité sympatho-vagale d'un individu comprenant un moyen de mesure d'une pluralité de mesures consécutives, dites mesures R-R, de l'intervalle de temps entre deux battements cardiaques de l'individu ou d'un animal de compétition, un dispositif selon la revendication 15 et un moyen de communication entre le moyen de mesure et ledit dispositif.

17. Système de détermination d'un indice représentatif de l'activité sympatho-vagale d'un individu ou d'un animal de compétition selon la revendication 16 dans lequel le moyen de mesure est un cardiofréquencemètre ou un textile instrumenté ou un électrocardiographe.

**Patentansprüche**

1. Computerimplementiertes Verfahren zur Bestimmung eines Indices, welcher die sympathovagale Aktivität eines Individuums oder eines Wettkampfstieres darstellt, wobei das Verfahren folgende Schritte umfasst:

- Empfangen (101) einer Sequenz, welche eine Vielzahl von Messungen aufweist, genannt R-R-Messungen, des Zeitintervalls zwischen zwei aufeinanderfolgenden Herzschlägen des Individuums oder des Wettkampfstieres, wobei das Verfahren **dadurch gekennzeichnet ist, dass** es ferner Folgendes umfasst:
- Bilden (105) einer ersten Teilsequenz, bestehend aus den positiven Abweichungen zwischen zwei aufeinanderfolgenden Werten der empfangenen Sequenz,
- Bilden (106) einer zweiten Teilsequenz, bestehend aus den Absolutwerten der negativen Abweichungen zwischen zwei aufeinanderfolgenden Werten der empfangenen Sequenz,
- Berechnen (107) eines Indices, welcher einem Wert gleich ist, welcher den Vergleich zwischen dem Ergebnis einer auf die Werte der ersten Teilsequenz angewandten statistischen Funktion und dem Ergebnis der auf die Werte der zweiten Teilsequenz angewandten statistischen Funktion darstellt.

2. Verfahren zur Bestimmung eines Indices, welcher die sympathovagale Aktivität eines Individuums oder eines Wettkampfstieres darstellt, nach Anspruch 1, wobei der berechnete Index gleich dem Verhältnis zwischen einer auf die Werte der ersten Teilsequenz angewandten statistischen Funktion und der auf die Werte der zweiten Teilsequenz angewandten statistischen Funktion ist.

3. Verfahren zur Bestimmung eines Indices, welcher die sympathovagale Aktivität eines Individuums oder eines Wettkampfstieres darstellt, nach Anspruch 2, wobei die statistische Funktion aus folgender Menge gewählt ist: einem Mittelwert, einer Varianz, einer Standardabweichung.

4. Verfahren zur Bestimmung eines Indices, welcher die sympathovagale Aktivität eines Individuums oder eines Wettkampfstieres darstellt, nach einem der vorhergehenden Ansprüche, ferner umfassend einen Schritt des Filterns (102) der empfangenen Sequenz, darin bestehend, Extremwerte und/oder Extremabweichungen zwischen zwei aufeinanderfolgenden Werten zu entfernen, welche aus Messartefakten resultieren und physiologisch unmöglichen Werten entsprechen.

5. Verfahren zur Bestimmung eines Indices, welcher die sympathovagale Aktivität eines Individuums oder eines Wettkampfstieres darstellt, nach Anspruch 4, wobei der Schritt des Filterns (102) mindestens die Entfernung derjenigen R-R-Messungen umfasst, deren Werte einen ersten Schwellenwert überschreiten oder einen zweiten Schwellenwert unterschreiten, wobei der erste und der zweite Schwellenwert gemäß der maximalen und der minimalen physiologisch möglichen Amplitude des Intervalls zwischen zwei Schlägen eines menschlichen Herzens oder eines Herzens eines Wettkampfstieres konfiguriert sind.

6. Verfahren zur Bestimmung eines Indices, welcher die sympathovagale Aktivität eines Individuums oder eines Wettkampfstieres darstellt, nach einem der Ansprüche 4 oder 5, wobei der Schritt des Filterns (102) mindestens die Entfernung einer R-R-Messung umfasst, wenn das Verhältnis zwischen dieser R-R-Messung und der vorhergehenden einen dritten Schwellenwert überschreitet oder einen vierten Schwellenwert unterschreitet, wobei der dritte Schwellenwert und der vierte Schwellenwert gemäß der physiologisch möglichen Höchst- und Mindestwerte des Verhältnisses konfiguriert sind.

7. Verfahren zur Bestimmung eines Indices, welcher die sympathovagale Aktivität eines Individuums oder eines Wettkampfstieres darstellt, nach einem der Ansprüche 4 bis 6, wobei der Schritt des Filterns (102) mindestens die Entfernung einer R-R-Messung umfasst, wenn:

    - die Abweichung zwischen dieser Messung und der vorhergehenden und die Abweichung zwischen dieser Messung und der darauffolgenden einen fünften Schwellenwert überschreiten, oder
    - die Abweichung zwischen dieser Messung und der vorhergehenden und die Abweichung zwischen dieser Messung und der darauffolgenden einen sechsten Schwellenwert unterschreiten,
    - wobei der fünfte Schwellenwert und der sechste Schwellenwert gemäß der physiologisch möglichen Höchst- und Mindestwerte der Abweichungen konfiguriert sind.

8. Verfahren zur Bestimmung eines Indices, welcher die sympathovagale Aktivität eines Individuums oder eines Wettkampfstieres darstellt, nach einem der Ansprüche 4 bis 7, wobei der Schritt des Filterns (102) mindestens die Entfernung einer R-R-Messung umfasst, wenn:

    - das Verhältnis zwischen dieser Messung und der vorhergehenden und das Verhältnis zwischen dieser Messung und der darauffolgenden einen siebten Schwellenwert überschreiten, oder
    - das Verhältnis zwischen dieser Messung und der vorhergehenden und das Verhältnis zwischen dieser Messung und der darauffolgenden einen achten Schwellenwert unterschreiten,
    - wobei der siebte Schwellenwert und der achte Schwellenwert gemäß der physiologisch möglichen Höchst- und Mindestwerte der Verhältnisse konfiguriert sind.

9. Verfahren zur Bestimmung eines Indices, welcher die sympathovagale Aktivität eines Individuums oder eines Wettkampfstieres darstellt, nach einem der Ansprüche 4 bis 8, wobei der Schritt des Filterns (102) mindestens die Entfernung einer R-R-Messung umfasst, wenn der Absolutwert der Abweichung zwischen dieser Messung und der vorhergehenden einen neunten Schwellenwert überschreitet, welcher von der über sämtliche Messungen der Sequenz berechneten Standardabweichung abhängt, wobei der neunte Schwellenwert gemäß der physiologisch möglichen Höchst- und Mindestwerte der Abweichung konfiguriert ist.

10. Verfahren zur Bestimmung eines Indices, welcher die sympathovagale Aktivität eines Individuums oder eines Wettkampfstieres darstellt, nach einem der Ansprüche 4 bis 9, wobei der Schritt des Filterns (102) mindestens die Entfernung einer R-R-Messung umfasst, wenn:

    - die Abweichung zwischen dieser Messung und der vorhergehenden und die Abweichung zwischen dieser Messung und der darauffolgenden einen zehnten Schwellenwert überschreiten, welcher von der über sämtliche Messungen der Sequenz berechneten Standardabweichung abhängt, oder
    - die Abweichung zwischen dieser Messung und der vorhergehenden und die Abweichung zwischen dieser Messung und der darauffolgenden einen elften Schwellenwert unterschreiten, welcher von der über sämtliche Messungen der Sequenz berechneten Standardabweichung abhängt,
    - wobei der zehnte Schwellenwert und der elfte Schwellenwert gemäß der physiologisch möglichen Höchst- und Mindestwerte der Abweichungen konfiguriert sind.

**11.** Verfahren zur Bestimmung eines Indices, welcher die sympathovagale Aktivität eines Individuums oder eines Wettkampfstieres darstellt, nach einem der vorhergehenden Ansprüche, ferner umfassend einen Schritt (104) vor der Bildung der ersten und der zweiten Teilsequenz, welcher darin besteht, eine vorbestimmte Anzahl an Messungen zu Beginn und am Ende der Sequenz dergestalt zu entfernen, dass Randeffekte korrigiert werden.

**12.** Verfahren zur Bestimmung eines Indices, welcher die sympathovagale Aktivität eines Individuums oder eines Wettkampfstieres darstellt, nach einem der vorhergehenden Ansprüche, wobei das Wettkampfstier ein Pferd ist.

**13.** Computerprogramm, aufweisend Anweisungen zur Ausführung des Verfahrens zur Bestimmung eines Indices, welcher die sympathovagale Aktivität eines Individuums oder eines Wettkampfstieres darstellt, nach einem der Ansprüche 1 bis 12, wenn das Programm von einem Prozessor ausgeführt wird.

**14.** Prozessorlesbarer Aufzeichnungsträger, auf welchem ein Computerprogramm nach Anspruch 13 aufgezeichnet ist.

**15.** Vorrichtung, umfassend einen Speicher und einen Prozessor, welche konfiguriert sind, um das Verfahren zur Bestimmung eines Indices, welcher die sympathovagale Aktivität eines Individuums oder eines Wettkampfstieres darstellt, nach einem der Ansprüche 1 bis 12 zu implementieren.

**16.** System zur Bestimmung eines Indices, welcher die sympathovagale Aktivität eines Individuums darstellt, das ein Mittel zur Messung einer Vielzahl von aufeinanderfolgenden Messungen, genannt R-R-Messungen, des Zeitintervalls zwischen zwei Herzschlägen des Individuums oder eines Wettkampfstieres, eine Vorrichtung nach Anspruch 15 und ein Kommunikationsmittel zwischen dem Messmittel und der Vorrichtung umfasst.

**17.** System zur Bestimmung eines Indices, welcher die sympathovagale Aktivität eines Individuums oder eines Wettkampfstieres darstellt, nach Anspruch 16, wobei das Messmittel ein Herzfrequenzmesser oder ein instrumentiertes Textil oder ein Elektrokardiograf ist.

**Claims**

**1.** A computer-implemented method for determining an index representing the sympathovagal activity of an individual or of a competition animal, said method comprising the following steps:

- receiving (101) a sequence with a plurality of measurements, called R-R measurements, of the time interval between two consecutive heartbeats of the individual or of the competition animal, the method being **characterised in that** it further comprises:
- constructing (105) a first sub-sequence made up of the positive deviations between two consecutive values of said received sequence;
- constructing (106) a second sub-sequence made up of the absolute values of the negative deviations between two consecutive values of said received sequence;
- computing (107) an index equal to a value representing the comparison between the result of a statistical function applied to the values of the first sub-sequence and the result of said statistical function applied to the values of the second sub-sequence.

**2.** The method for determining an index representing the sympathovagal activity of an individual or of a competition animal according to claim 1, wherein the computed index is equal to the ratio between a statistical function applied to the values of the first sub-sequence and said statistical function applied to the values of the second sub-sequence.

**3.** The method for determining an index representing the sympathovagal activity of an individual or of a competition animal according to claim 2, wherein said statistical function is taken from the following set: an average, a variance, a standard deviation.

**4.** The method for determining an index representing the sympathovagal activity of an individual or of a competition animal according to any of the preceding claims, further comprising a step (102) of filtering said received sequence consisting in removing extreme values and/or extreme deviations between two consecutive values, resulting from measurement artefacts and corresponding to physiologically impossible values.

**5.** The method for determining an index representing the sympathovagal activity of an individual or of a competition animal according to claim 4, wherein the filtering step (102) comprises at least the deletion of the R-R measurements for which the values are higher than a first threshold or lower than a second threshold, said first and second thresholds being configured as a function of the maximum and minimum physiologically possible amplitude of the interval between two beats of a human heart or of a heart of a competition animal.

6. The method for determining an index representing the sympathovagal activity of an individual or of a competition animal according to any of claims 4 or 5, wherein the filtering step (102) comprises at least the deletion of an R-R measurement if the ratio between this R-R measurement and the preceding measurement is higher than a third threshold or is lower than a fourth threshold, said third threshold and said fourth threshold being configured as a function of the maximum and minimum physiologically possible values of said ratio.

7. The method for determining an index representing the sympathovagal activity of an individual or of a competition animal according to one of claims 4 to 6, wherein the filtering step (102) comprises at least the deletion of an R-R measurement if:

   - the deviation between this measurement and the preceding measurement and the deviation between this measurement and the next measurement are higher than a fifth threshold; or
   - the deviation between this measurement and the preceding measurement and the deviation between this measurement and the next measurement are lower than a sixth threshold;
   - said fifth threshold and said sixth threshold being configured as a function of the maximum and minimum physiologically possible values of said deviations.

8. The method for determining an index representing the sympathovagal activity of an individual or of a competition animal according to one of claims 4 to 7, wherein the filtering step (102) comprises at least the deletion of an R-R measurement if:

   - the ratio between this measurement and the preceding measurement and the ratio between this measurement and the next measurement are higher than a seventh threshold; or
   - the ratio between this measurement and the preceding measurement and the ratio between this measurement and the next measurement are lower than an eighth threshold;
   - said seventh threshold and said eighth threshold being configured as a function of the maximum and minimum physiologically possible values of said ratios.

9. The method for determining an index representing the sympathovagal activity of an individual or of a competition animal according to one of claims 4 to 8, wherein the filtering step (102) comprises at least the deletion of an R-R measurement if the absolute value of the deviation between this measurement and the preceding measurement is higher than a ninth threshold dependent on the standard deviation computed over all the measurements of the sequence, said ninth threshold being configured as a function of the maximum and minimum physiologically possible values of said deviation.

10. The method for determining an index representing the sympathovagal activity of an individual or of a competition animal according to one of claims 4 to 9, wherein the filtering step (102) comprises at least the deletion of an R-R measurement if:

   - the deviation between this measurement and the preceding measurement and the deviation between this measurement and the next measurement are higher than a tenth threshold dependent on the standard deviation computed over all the measurements of the sequence; or
   - the deviation between this measurement and the preceding measurement and the deviation between this measurement and the next measurement are lower than an eleventh threshold dependent on the standard deviation computed over all the measurements of the sequence;
   - said tenth threshold and said eleventh threshold being configured as a function of the maximum and minimum physiologically possible values of said deviations.

11. The method for determining an index representing the sympathovagal activity of an individual or of a competition animal according to one of the preceding claims, further comprising a step (104), prior to the construction of the first and of the second subsequences, involving deleting a predetermined number of measurements at the start and at the end of the sequence so as to correct the edge effects.

12. The method for determining an index representing the sympathovagal activity of an individual or of a competition animal according to one of the preceding claims, wherein the competition animal is a horse.

13. A computer program having instructions for executing the method for determining an index representing the sympathovagal activity of an individual or of a competition animal according to one of claims 1 to 12, when the program is executed by a processor.

14. A processor-readable recording medium, on which a computer program according to claim 13 is recorded.

15. A device comprising a memory and a processor configured to implement the method for determining an index representing the sympathovagal activity of an individual or of a competition animal according to one of claims 1 to 12.

16. A system for determining an index representing the sympathovagal activity of an individual comprising a means for measuring a plurality of consecutive measurements, called R-R measurements, of the time interval between two heartbeats of the individual or of a competition animal, a device according to claim 15 and a means for communicating between the measurement means and said device.

17. A system for determining an index representing the sympathovagal activity of an individual or of a competition animal according to claim 16, wherein the measurement means is a heart rate monitor or an instrumented textile or an electrocardiograph.

101 — Acquisition mesures R-R

102 — Filtrage artefacts mesures

103 — Validation filtrage — non

oui

104 — Suppression effets de bords

105 — Construction sous-séquence 1

106 — Construction sous-séquence 2

107 — Calcul indice

FIG.1

FIG.2a

EP 3 017 754 B1

FIG.2b

FIG.3

401 — Détermination de plusieurs indices de forme sur une durée donnée

402 — Normalisation

403 — Interpolation 1

404 — Interpolation 2

405 — Filtrage

# FIG.4a

$LI_0$

$LI_1$

$LI_2$

$LI_3$

I

# FIG.4b

EP 3 017 754 B1

FIG.5

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- Heart rate variability. Standards of measurement, physiological interprétation and clinical use. *American Heart Association Inc.: European Society of Cardiology,* 1996
- **DAMIEN SABOUL et al.** The impact of breathing on HRV measurements: Implications for the longitudinal follow-up of athletes. *European Journal of Sport Science,* 2013
- **DANIEL J. PLEWS.** Training adaptation and heart rate variability in elite endurance athletes: opening the door to effective monitoring. Springer International Publishing, 2013

- **FERDINANDO LELLAMO.** Conversion from vagal to sympathetic prédominance with strenuous training in high performance world class athletes. American Heart Association, 2002
- **DWAIN L. ECKBERG.** Sympathovagal Balance, a critical appraisal. American heart association, inc, 1997
- **DAMIEN SABOUL et al.** The breathing effect of the LF/HF ratio in the heart rate variability measurements of athletes. *European Journal of sport science,* 2012
- **GUZIK et al.** Correlations between the Poincaré Plot and conventional heart rate variability parameters assessed during paced breathing. *J.Physiol. Sci.,* Février 2007, vol. 57 (1), 63-71